# EUROPÄISCHE PATENTSCHRIFT

(11) **EP 3 743 130 B1**
(45) Veröffentlichungstag und Bekanntmachung des Hinweises auf die Patenterteilung: **25.03.2026**
(21) Anmeldenummer: 19701061.4
(22) Anmeldetag: 15.01.2019
(51) Int. Cl.: A61M 60/148, A61M 60/191, A61M 60/289, A61M 60/839, A61M 60/861

(54) **HERZUNTERSTÜTZUNGSVORRICHTUNG MIT STRUKTURIERTER OBERFLÄCHE**
VENTRICULAR ASSIST DEVICE WITH STRUCTURED SURFACE
DISPOSITIF D'ASSISTANCE VENTRICULAIRE À SURFACE TEXTURÉE

(30) Priorität: 23.01.2018 DE 102018200974
(43) Veröffentlichungstag der Anmeldung: 02.12.2020
(73) Patentinhaber: AdjuCor GmbH, 81673 München (DE)
(72) Erfinder: MAIER, Andreas, 85567 Grafing b. München (DE); HOFMANN, Björn, 85567 Bruck (DE); DE VAAL, Hamman, Cape Town, 8001 (ZA); M. WILDHIRT, Stephen, 82335 Berg (DE)
(74) Vertreter: Peterreins Schley
(86) Internationale Anmeldenummer: PCT/EP2019/050894
(87) Internationale Veröffentlichungsnummer: WO 2019/145189

(56) Entgegenhaltungen:
- EP-A1- 2 752 209
- EP-A1- 3 115 023
- WO-A1-2015/142753
- WO-A1-90/10782
- WO-A1-94/02101
- WO-A2-2004/008941
- WO-A2-2010/135437
- US-A- 3 843 974
- US-A- 5 685 306
- US-A1- 2006 064 159

## Beschreibung

### Technisches Gebiet

Die vorliegende Erfindung betrifft eine Herzunterstützungsvorrichtung umfassend ein Implantat mit einer Hülle, wobei die Hülle eine strukturierte Oberfläche hat, die das Einwachsen von Bindegewebe fördert.

### Hintergrund der Erfindung

Krankheitsbedingt kann die Pumpfunktion eines Herzens reduziert sein, was auch als Herzinsuffizienz bezeichnet wird. Die Herzinsuffizienz ist sowohl aus medizinischer als auch aus ökonomischer Sicht von großer und wachsender Bedeutung. In der zweiten Dekade dieses Jahrhunderts werden weltweit 23 Mio. Menschen an der Herzinsuffizienz leiden, die jährliche Neuerkrankungsrate wird dann bei 2 Mio. Menschen liegen. Alleine in den USA sind derzeit ca. 5 Mio. Menschen an Herzinsuffizienz erkrankt. Hier beträgt die jährliche Neuerkrankungsrate ca. 550.000 Menschen. Bereits in diesem Jahrzehnt werden sich alleine in den USA die Zahlen bei den über 50-Jährigen auf über 10 Mio. verdoppeln. Das Gleiche gilt für den europäischen Kontinent.

Ursächlich für eine Herzinsuffizienz kann eine verschlechterte Kontraktionsfähigkeit oder Füllung des Herzens durch eine Schädigung des Herzmuskels (Myokards) sein. Ein erhöhter Blutdruck kann zu einem erhöhten Pumpwiderstand führen, der sich ebenfalls negativ auf die Pumpfunktion des Herzens auswirken kann. Die Pumpfunktion eines Herzens kann auch durch undichte Klappen, wie z.B. einer undichten Aortenklappe oder Mitralklappe, vermindert sein.

Verschiedene Arten der Herzinsuffizienz können medikamentös oder operativ behandelt werden. Neben weiteren Behandlungsmethoden stellt die Unterstützung der Pumpfunktion des Herzens durch ein Implantat, das auf das Herz einen mechanischen Druck ausübt und damit dessen Pumpleistung verbessert, einen vielversprechenden, die verschiedenen Ursachen der Herzinsuffizienz übergreifenden Ansatz der Behandlung dar.

Eine Herzunterstützungsvorrichtung kann aus einem Implantat mit einer Hülle bestehen. Auf der Hülle oder in der Hülle können expandierbare Einheiten angebracht sein, mit Hilfe derer ein mechanischer Druck auf das Herz ausgeübt werden kann. Die Herzunterstützungsvorrichtung kann eine Versorgungseinheit umfassen, die mit dem Implantat verbunden ist. Eine Herzunterstützungsvorrichtung mit einer selbst-expandierenden Schale ist unter anderem in der EP 2 752 209 A1 beschrieben.

Eine mechanische Herzunterstützungsvorrichtung kann auch ohne expandierbare Einheiten ausgestaltet sein und damit eine passive Herzunterstützungsvorrichtung sein. Passive Herzunterstützungsvorrichtungen sind auch als Herzband, Herzharnisch, diastolic recoil device oder cardiac harness bekannt. Eine passive Herzunterstützung ist unter anderem in EP 2 456 483 B1 beschrieben.

EP 3 115 023 A1 offenbart eine Vorrichtung zur Applikation von Substanzen auf die epikardiale Herzoberfläche, wobei die Vorrichtung eine Rahmenstruktur und eine Hülle enthält.

Aufgabe der vorliegenden Erfindung ist es, die Biokompatibilität des Implantats zu verbessern. Eine weitere Aufgabe ist es, eine Hülle bereitzustellen, die ein geeignetes Widerlager für eine Herzunterstützungsvorrichtung bildet.

### Zusammenfassung der Erfindung

Die vorliegende Erfindung betrifft eine wie in den Ansprüchen definierte Herzunterstützungsvorrichtung. Die erfindungsgemäße Herzunterstützungsvorrichtung umfasst ein Implantat mit einer Hülle, wobei die Hülle eine innere Oberfläche und eine äußere Oberfläche hat. Die innere Oberfläche ist herzzugewandt und ausgelegt, im implantierten Zustand das Epikard zu berühren. Die äußere Oberfläche ist herzabgewandt und ausgelegt, im implantierten Zustand das Perikard zu berühren. Die innere und die äußere Oberfläche der Hülle haben eine strukturierte Oberfläche, die das Einwachsenvon Bindegewebe fördert. Die Struktur, die das Einwachsen von Gewebe fördert kann eine poröse und/oder eine raue Struktur sein. Die Vorrichtung kann weiterhin zumindest eine expandierbare Einheit umfassen, die eine Kraft auf das Herz ausüben kann. Die zumindest eine expandierbare Einheit kann eine strukturierte Oberfläche aufweisen, welche das Einwachsen von Gewebe fördert. Die Vorrichtung kann auch ohne eine expandierbare Einheit ausgestaltet sein und damit eine passive Herzunterstützungsvorrichtung sein. Die Herzunterstützungsvorrichtung kann eine aktive Herzunterstützungsvorrichtung sein und zumindest eine expandierbare Einheit und/oder zumindest eine Elektrode umfassen

Die Oberfläche der Hülle kann eine poröse Oberfläche umfassen. Die Oberfläche der Hülle kann Poren umfasst, wobei die Poren eine Größe von 1 µm bis 1000 µm, insbesondere von 10 µm bis 500 µm umfassen. Vorzugsweise haben die Poren eine Größe von 40 µm bis 300 µm. Die Poren können das Einwachsen von Gewebe, insbesondere von Bindegewebe, fördern.

Die Oberfläche der Hülle kann eine raue Oberfläche umfassen. Die raue Oberfläche kann eine Rauigkeit von 1 µm bis 1000 µm, insbesondere von 10 µm bis 500 µm aufweisen. Vorzugsweise hat die raue Oberfläche eine Rauigkeit von 40 µm bis 300 µm.

Die Oberfläche der Hülle kann eine Oberflächenbeschichtung umfassen. Die Oberflächenbeschichtung kann ein poröses Material umfassen. Das poröse Material kann eine geschäumte Schicht, eine aus kleinen Festkörpern gepackte Schicht, eine Wabenstruktur, oder Fasern umfassen. Die Oberflächenbeschichtung kann eine raue Schicht umfassen.

Alternativ oder zusätzlich kann die raue und/oder poröse Oberfläche der Hülle durch eine Oberflächenumwandlung erzielt werden. Die dadurch entstehende Oberfläche kann die zuvor beschriebenen Porengrößen / Rauigkeiten aufweisen. Alternativ oder zusätzlich kann eine raue und/oder poröse Oberfläche auch urformend hergestellt werden.

Eine derartige Porosität/Rauigkeit kann das Einwachsen von Gewebe, insbesondere von Bindegewebe, fördern. Eine poröse oder raue Strukturierung der Hülle des Implantats kann dazu führen, dass die große flächenhafte Oberfläche des Implantats von den Makrophagen oder Riesenzellen nicht mehr als ununterbrochene kontinuierliche Oberfläche erkannt wird. Festkörperstrukturen zwischen den Hohlräumen einer porösen Strukturierung oder Berg-und-Tal einer rauen Strukturierung können von den Makrophagen und Riesenzellen als ausreichend kleiner Fremdkörper wahrgenommen werden, so dass eine Einkapselung und das Einwachsen von Bindegewebe direkt an der Oberfläche der Hülle erfolgreich abgeschlossen werden kann. Die Oberfläche der Hülle kann daher eine poröse oder raue Oberfläche aufweisen, bei der sich die Porengröße oder die Rauigkeit im Bereich zwischen 1 µm und 1000 µm, 10 µm bis 500 µm insbesondere zwischen 40 µm und 300 µm befindet.

Die Erfindung betrifft auch Verfahren zum Herstellen einer Herzunterstützungsvorrichtung umfassend ein Implantat mit einer Hülle, wobei die Hülle eine strukturierte Oberfläche hat, die das Einwachsen von Gewebe, insbesondere von Bindegewebe, fördert. Ein Verfahren kann das Bereitstellen einer Hülle und das Anbringen einer porösen oder rauen Beschichtung auf die Oberfläche der Hülle umfassen. Ein Verfahren kann alternativ oder zusätzlich das Bereitstellen eines Implantats mit einer Hülle und das Umwandeln der Oberfläche der Hülle umfassen, wobei eine poröse oder raue Oberfläche entsteht.

### Übersicht der Figuren

**Figur 1** zeigt eine Ausführungsform eines Implantats einer Herzunterstützungsvorrichtung.
**Figur 2** zeigt einen Schnitt durch ein Herz mit einer Hülle und einer expandierbaren Einheit
**Figur 3** zeigt die Kaskade der Einkapselung
**Figur 4a****-b** zeigen einen Schnitt durch das Herz mit Wucherung um das Myokard, verursacht durch eine nicht biokompatible Hülle
**Figur 5a****-b** zeigen einen Schnitt durch ein Herz mit einer eingekapselten Hülle ohne Wucherung um Myokard oder Perikard
**Figur 6a****-b** zeigt einen Schnitt durch eine implantierte Hülle mit erfindungsgemäßer strukturierter Oberfläche
**Figur 7a****-b** zeigen eine Bild bzw. eine schematische Darstellung von einem Schnitt durch eine erfindungsgemäße Hülle mit einer porösen und einer rauen Oberfläche

### Detaillierte Beschreibung

Die vorliegende Erfindung betrifft eine wie in den Ansprüchen definierte Herzunterstützungsvorrichtung. Erfindungsgemäße Ausführungsformen werden im Folgen unter Bezugnahmen auf die Figuren näher beschreiben.

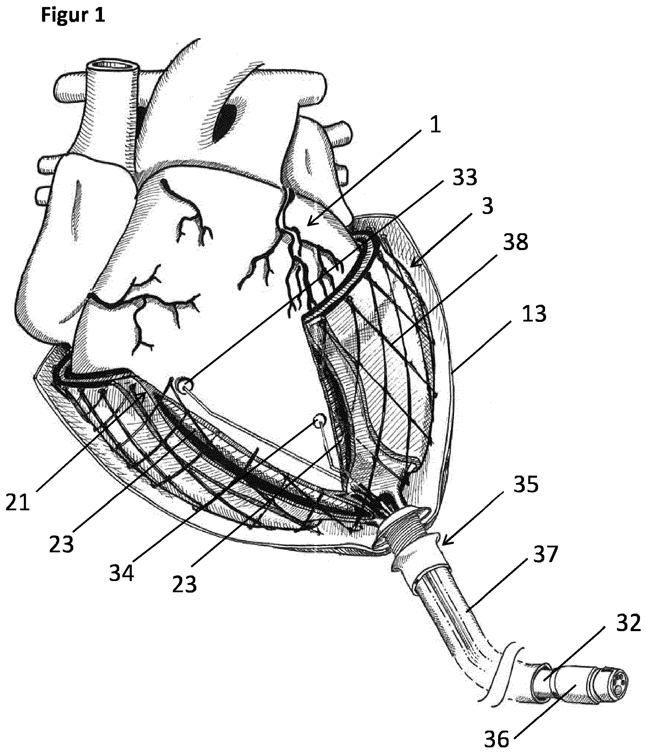

**Figur 1** zeigt exemplarisch ein menschliches Herz (1) sowie eine Schale (38), eine Hülle (21) mit expandierbaren Einheiten (23), Sensoren (33) und/oder Elektroden (34), ein Kabel (32) mit einem Steckerteil (36), einen Katheter (37) eines Zuführsystems, ein Perikard (13) und einen Perikardverschluss (35). Die dargestellte Schale besteht aus einem Drahtgeflecht. Eine Schale (38) ist jedoch für die Funktion der Herzunterstützungsvorrichtung (2) nicht zwingend erforderlich und kann auch weggelassen werden. Insbesondere wenn eine Hülle (21) verwendet wird, die selbst ein geeignetes Widerlager bilden kann, kann auf die Schale (38) verzichtet werden.

Die Hülle (21) ist vorzugsweise derart ausgestaltet, dass sie das Herz zumindest teilweise umschließen kann. Die Hülle bedeckt vorzugsweise die untere Hälfte des Herzens. Weiterhin ist es vorteilhaft, wenn die Hülle und/oder die expandierbaren Einheiten der Hülle sich nicht über die Klappenebene des Herzens erstreckt. Expandierbare Einheiten auf Höhe der Herzklappenebene können unter Umständen zur Beeinträchtigung der Klappenfunktionen führen. Weiterhin kann die Hülle Aussparungen aufweisen, um sich besser der Anatomie des Herzens anzupassen. Beispielsweise kann die Hülle eine Aussparung für die Vena cava haben. Damit wird eine bessere Passform erreicht und die Vena cava nicht berührt. Wenn die Vena cava kompromittiert wird, kann es zu einer Einflussstauung führen.

In der Figur 1 werden zahlreiche Elemente wie zum Beispiel expandierbare Einheiten (23), Sensoren (33), Elektroden (34), Kabel (32), Katheter (37), etc. gezeigt, die ebenfalls nicht erfindungswesentlich sind. Eine Herzunterstützungsvorrichtung (2) ohne eine expandierbare Einheit (23) ist eine passive Herzunterstützungsvorrichtung (2) (auch bekannt als Herzband, Herzharnisch, diastolic recoil device oder cardiac harness) und kann die Bewegung des Herzens (1) während der Pumpaktivität, beispielsweise die Kontraktion während Auswurfphase (Systole) oder die Ausdehnung des Herzens (1) während der Füllung (Diastole) oder in Folge von Wachstum, einschränken. Eine aktive Herzunterstützungsvorrichtung (2) kann expandierbare Einheiten (23) umfassen mit Hilfe derer eine Kraft auf die Herzwand ausgeübt werden kann, sodass die Pumpfunktion des Herzens (1) in der Systole und/oder Diastole unterstützt wird. Eine aktive Herzunterstützungsvorrichtung (2) kann Elektroden (34) umfassen, mit Hilfe derer das EKG des Herzens (1) zur zeitlichen optimierten Ansteuerung der Herzunterstützungsvorrichtung (2) gemessen und/oder das Herz (1) zur Unterstützung der Pumpfunktion mit elektrischem Strom stimuliert werden. Es ist auch möglich, expandierbare Einheiten (23) und Elektroden (34) zu verwenden, um die Pumpfunktion des Herzens (1) zu verbessern.

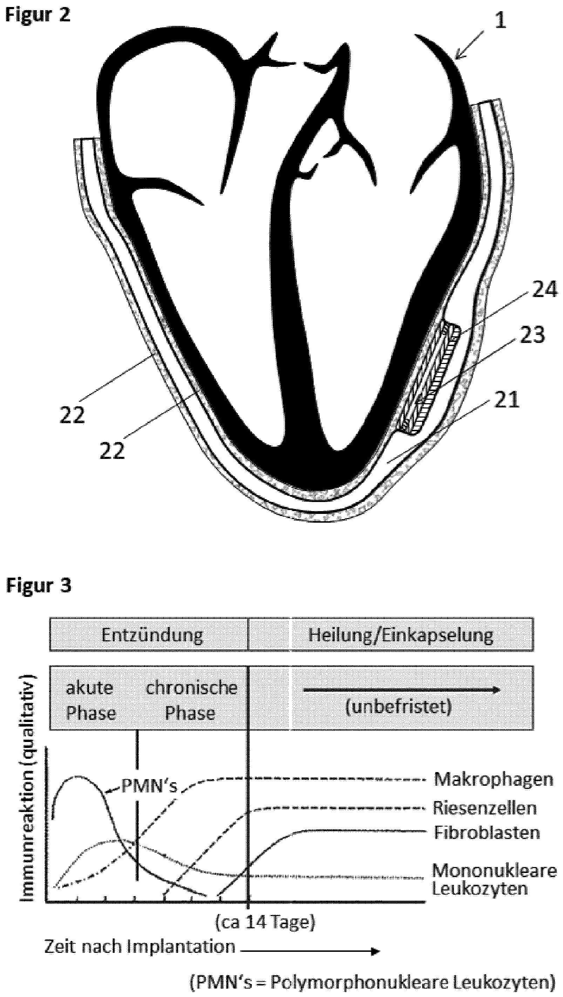

**Figur 2** zeigt einen Schnitt durch ein Herz (1) mit einer erfindungsgemäßen Hülle (21), die im dargestellten Beispiel eine expandierbare Einheit (23) umfasst. Die zumindest eine expandierbare Einheit (23) kann auf einer beliebigen Stelle der Außen- oder Innenwand der Hülle (21) positioniert werden. Die zumindest eine expandierbare Einheit (23) kann Bestandteil der Hülle (21) sein. Die Hülle (21) kann einen Teil der expandierbaren Einheit (23) formen. Die zumindest eine expandierbare Einheit (23) kann auch auf der Hülle (21) mittels Formschluss, Kraftschluss und/oder Stoffschluss befestigt werden. Die Figur zeigt beispielhaft eine expandierbare Einheit (23), die mit einer Klebeverbindung (24) auf der Innenwand der Hülle (21) angebracht ist. Die Hülle (21) hat eine innere, herzzugewandte Oberfläche und eine äußere, herzabgewandte Oberfläche. Erfindungsgemäß hat die innere und die äußere Oberfläche der Hülle (21) eine strukturierte Oberfläche (22), die das Einwachsen von Bindegewebe fördert. Die optionale expandierbare Einheit (23) kann ebenfalls eine strukturierte Oberfläche (22) aufweisen, die das Einwachsen von Gewebe, insbesondere von Bindegewebe, fördert.

Eine strukturierte Oberfläche (22), die das Einwachsen von Gewebe fördert, kann eine poröse oder eine raue Oberfläche sein. Eine poröse oder eine raue Oberfläche (22) der Hülle (21) und/oder einer expandierbaren Einheit (23) kann urformend hergestellt werden. Weiter kann eine Hülle (21) und/oder eine expandierbare Einheit (23) mit einer porösen oder rauen Beschichtung versehen werden, um eine strukturierte Oberfläche (22) auf der Hülle (21) und/oder der expandierbaren Einheit (23) zu erzeugen. Weiter kann die Oberfläche der Hülle (21) und/oder einer expandierbaren Einheit (23) derart durch Oberflächenumwandlung, beispielsweise durch chemische oder physikalische Oberflächenumwandlung, modifiziert werden, sodass eine poröse oder raue Oberfläche (22) entsteht.

Das Einwachsen von Gewebe, insbesondere von Bindegewebe, ist unter mechanischen Gesichtspunkten vorteilhaft. Durch das Einwachsen von Gewebe wird die Steifigkeit der Hülle (21) erhöht. Dadurch entsteht ein gutes Widerlager sowohl für aktive wie auch passive Herzunterstützungsvorrichtungen. Im Falle einer aktiven Herzunterstützungsvorrichtung bildet die durch Gewebe versteifte Hülle (21) ein optimales Widerlager für die expandierbaren Einheiten (23). Im Falle einer passiven Herzunterstützungsvorrichtung bildet die durch Gewebe versteifte Hülle (21) ein Widerlager, das die Ausdehnung des Herzens (1) beschränkt. Die Erhöhung der Steifigkeit der Hülle (21) durch das Einwachsen ist darauf zurückzuführen, dass Gewebe in das poröse oder raue Material der strukturierten Oberfläche (22) einwächst und die strukturierte Oberfläche (22) in Kombination mit dem Gewebe besser mechanische Kräfte aufnehmen kann. Die Dicke der mechanisch belastbaren Anteile der Hülle (21) wird somit größer. Die Dicke bzw. die Höhe der strukturierten Oberfläche (22), kann 1 µm bis hin zu 5 mm betragen, bevorzugt 40 µm bis 3 mm. Wenn kein Gewebe in das raue oder poröse Material der strukturierten Oberfläche (22) einwachsen würde, so würde das Material der strukturierten Oberfläche (22) deutlich weniger mechanische Last aufnehmen oder abtragen können.

Ein weiterer Vorteil einer Hülle (21) mit strukturierter Oberfläche (22) gegenüber einer Hülle (21) mit unstrukturierter Oberfläche ist beispielsweise, dass das kontinuierliche Kernmaterial der strukturierten Hülle (21) dünner gestaltet werden kann als bei einer Hülle (21) mit unstrukturierter Oberfläche um ein gleichwertiges Widerlager zu bieten.

Weiter hat eingewachsenes Gewebe gegenüber einem kontinuierlichem Material, beispielsweise einem Kunststoff, überlegenen mechanische Eigenschaften bezüglich Zug-Druck-Belastung. Gewebe reagieren allgemein sehr weich, nachgiebig und/oder flexibel auf Druckbelastung, aber steif auf Zugbelastung. Beispielsweise wird eine Hülle (21), die mit Gewebe eingewachsen ist und damit mit dem Herz (1) verbunden ist, bei der Kontraktion des Herzens (1) mitbewegt und damit zusammengedrückt. Auf diese Druckbelastung reagiert das eingewachsene Gewebe sehr weich, nachgiebig und/oder flexibel. Die gesamte Hülle (21) mit strukturierter Oberfläche (22) und das eigewachsene Gewebe verhalten sich somit während der Kontraktion des Herzens (1) sehr weich und flexibel und behindern damit die Kontraktion und die systolische Bewegung des Herzens (1) nicht. Würde sich hingegen ein Herz (1) zu sehr vergrößern, beispielsweise durch übermäßige Füllung oder durch krankheitsbedingte Ausdehnung des Myokards, so würden die Hülle (21) und das eingewachsene Gewebe gedehnt werden. Das somit unter Zugbelastung beanspruchte eingewachsene Gewebe reagiert steif und kann der Ausdehnung des Myokards Kräfte entgegensetzen. Kontinuierliche Kunststoffe hingegen würden auf Druck- und Zugbelastung ähnlich steif reagieren und können die natürliche Herzbewegung, insbesondere in der Systole behindern und die Auswurfleistung des Herzens (1) senken.

Weiter kann das Einwachsen von Gewebe eine Fixierung der Hülle (21) relativ zum Herzen (1) ermöglichen, womit eine unbeabsichtigte Dislokation des Implantats verhindert wird. Eine Dislokation des Implantats kann dazu führen, dass die Herzunterstützungsvorrichtung keine das Herz (1) unterstützende Funktion mehr ausüben kann.

Ein weiterer Vorteil einer Hülle (21), die das Einwachsen von Gewebe, insbesondere von Bindegewebe, fördert ist, dass Ungenauigkeiten in der Passform der Hülle (21) zum individuellen Patientenherz (1) durch das Einwachsen von Gewebe kompensiert werden kann. Beispielsweise wird eine raue oder poröse Strukturierung (22) bei der Implantation der erfindungsgemäßen Vorrichtung an Stellen, wo das Implantat lokal enger am Herzen (1) sitzt, zusammengedrückt. An solcher Stelle würde auch das Gewebe dann dünner einwachsen. Hingegen kann sich eine raue oder poröse Strukturierung (22) auf der erfindungsgemäßen Vorrichtung, an Stellen wo das Implantat lokal nicht zu eng am Herzen (1) sitzt, bis zu seiner gefertigten Dicke bzw. Höhe ausdehnen und so Zwischenräume zwischen dem Epi-/Myokard des Herzens (1) und dem kontinuierlichen Kernmaterial einer Hülle (21) und/oder einer expandierbaren Einheit (23) nach Implantation auffüllen. Das Gewebe würde an solchen Stellen bis hin zu der vollen Dicke der strukturierten Oberfläche (22) in die Strukturierungsschicht (22) einwachsen. Es würde so selektiv lokal unterschiedlich dick Gewebe in die strukturierte Oberfläche (22) einwachsen. Beispielsweise können so Fertigungsungenauigkeiten kompensiert werden. Beispielsweise können durch eine strukturierte Oberfläche (22) auf der Innenseite des Implantats, bei einer Höhe der Strukturierung von mehr als 0,5 mm, 1 mm, 2 mm, 3 mm, 4 mm, oder 5 mm auch Fertigungsungenauigkeiten, beispielsweise Unebenheiten, von 0,5 mm, 1 mm, 2 mm, 3 mm, 4 mm, oder 5 mm kompensiert werden, ohne dass das Implantat seine Passform zum Herzen (1) verliert. Beispielsweise können strukturierte Oberflächen (22), welche das Einwachsen mit Gewebe fördern, dazu eingesetzt werden, um durch selektives Einwachsen mit Gewebe eine patientenspezifische Passform zu erreichen, ohne dass eine vollständig patientenspezifische Anpassung der Implantatsform während des Fertigungsprozesses erforderlich ist. Eine zu hohe Strukturierung auf der Innenseite einer Hülle (21) eines aktiven Herzunterstützungssystems kann die Unterstützung der Pumpfunktion verschlechtern, da Gewebe in die Strukturierung und somit zwischen die Hülle (21) und das Epi-/Mykoard einwächst. Es kann daher auch nur eine dünne Strukturierung auf der Innenseite einer Hülle (21) angebracht werden, beispielsweise mit einer Höhe der Strukturierung (22) zwischen 10 µm bis 3 mm, insbesondere zwischen 40 µm bis 2 mm. Durch eine dünne Strukturierung (22) der Innenseite eines Implantats kann ein hoher Wirkungsgrad der Herzunterstützung erreicht werden. Es können auch unterschiedlich hohe Strukturierungen (22) auf der Innenseite einer Hülle (21) und der Außenseite einer Hülle (1) angebracht werden. Eine hohe Strukturierung (22) auf der Außenseite, beispielsweise von mehr als 1 mm, 2 mm, 3 mm, 4 mm oder 5 mm, lässt mehr Gewebe einwachsen und kann mechanisch ein besseres Widerlager für beispielsweise eine expandierbare Einheit (23) darstellen. Eine dünne Strukturierung (22) auf der Außenseite einer Hülle (21), beispielsweise von weniger als 3 mm, 2 mm, 1 mm, 0,5 mm oder 0,1 mm, kann jedoch auch den Vorteil haben, dass weniger Gewebe zwischen Implantat und Perikard einwächst, das Implantat damit kleinere Dimensionen aufweist und das Perikard weniger ausdehnt.

Ein weiterer Vorteil einer Hülle (21), die das Einwachsen von Gewebe, insbesondere von Bindegewebe, fördert ist, dass diese eine bessere Biokompatibilität aufweist. Diese und weitere Vorteile werden dadurch erreicht, dass eine Hülle (21) bereitgestellt wird, die eine poröse oder raue Oberfläche (22) aufweist.

Das Vorhandensein einer porösen oder rauen Oberfläche (22) kann die Biokompatibilität des Implantats und die Immunreaktion des Patientenkörpers bezüglich des Implantats beeinflussen. Vereinfacht dargestellt, gibt es im menschlichen Körper verschiedene Reaktionen des Immunsystems auf einen erkannten implantierten Fremdkörper. Diese sind (i) der Abbau des Fremdkörpers, (ii) das Absterben des Gewebes um den Fremdkörper herum, was insbesondere bei toxischen Fremdkörpern der Fall ist, und (iii) die Einkapselung des Fremdkörpers.

Ein Abbau des Materials ist bei der erfindungsgemäßen Vorrichtung nicht erwünscht. Durch geeignete Wahl der Werkstoffe kann erreicht werden, dass das Implantat nicht innerhalb eines vorgesehenen Zeitraumes abgebaut wird. Beispielsweise sind viele Silikone und einige Polyurethane und Polyester stabil gegen hydrolytische Spaltung oder oxidativen Abbau. Für die erfindungsgemäße Vorrichtung ist es weiterhin erwünscht, dass die Materialien oder gegebenenfalls entstehende Abbauprodukte oder herauslösbare Bestandteile nicht toxisch sind.

Nach Implantation der erfindungsgemäßen Vorrichtung versucht der Körper, das Implantat, das sich zumindest teilweise um die Ventrikel eines Herzens befindet, einzukapseln. Die dabei ablaufende Kaskade ist in **Figur 3** dargestellt, die von Szycher (Szycher's Handbook of Polyurethanes, Second Edition, CRC Press, 2013) adapiert wurde. Bei der Einkapselung läuft zunächst eine akute Entzündungsphase ab, in der Leukozyten präsent sind, welche dann eine zunehmende Makrophageneinwanderung hervorrufen. In einer anschließenden chronischen Entzündungsphase reduzieren sich Leukozyten, während eine weiterhin hohe Makrophageneinwanderung mit beginnender Riesenzellenbildung stattfindet. Makrophagen und Riesenzellen sind für die Einkapselung oder das Einschließen ("Phagocytose") eines Fremdkörpers zuständig (Makrophagen für Fremdkörper < 10 µm, Riesenzellen für Fremdkörper mit ca. 10-100 µm). Makrophagen aktivieren Fibroblasten. Gegen Ende der chronischen Phase produzieren diese Fibroblasten daher Bindegewebe, insbesondere Kollagen, welches als Matrixgewebe für einen Heilungsprozess verstanden werden kann und was als Abschluss der Kaskade gilt.

Weist ein Fremdkörper eine unstrukturierte Oberfläche auf und ist er zu groß, so dass eine Riesenzelle diesen nicht einschließen kann, kann dies zu einer frustrierten Phagocytose führen. Es kommt zu einer Anhäufung von Riesenzellen, welche weiter proinflammatorische Cytokine sekretieren, welche weitere Makrophagen rekrutieren und Riesenzellen entstehen lassen. Dies kann als Immunsystemüberreaktion verstanden werden, wobei einerseits an der Oberfläche des Implantats der Entzündungsprozess immer wieder von vorne entfacht wird, hingegen in implantatsferneren Bereichen der Entzündungsprozess abgeschlossen ist und Kollagenschichten und Granulationsgewebe vorliegen Bereits über Zeiträume von Tagen oder über eine längere Zeitdauer können sich so millimeter- oder zentimeterdicke Schichten aus Kollagen und/oder Granulationsgewebe aufbauen, welche sich beispielsweise als Wucherung darstellen.

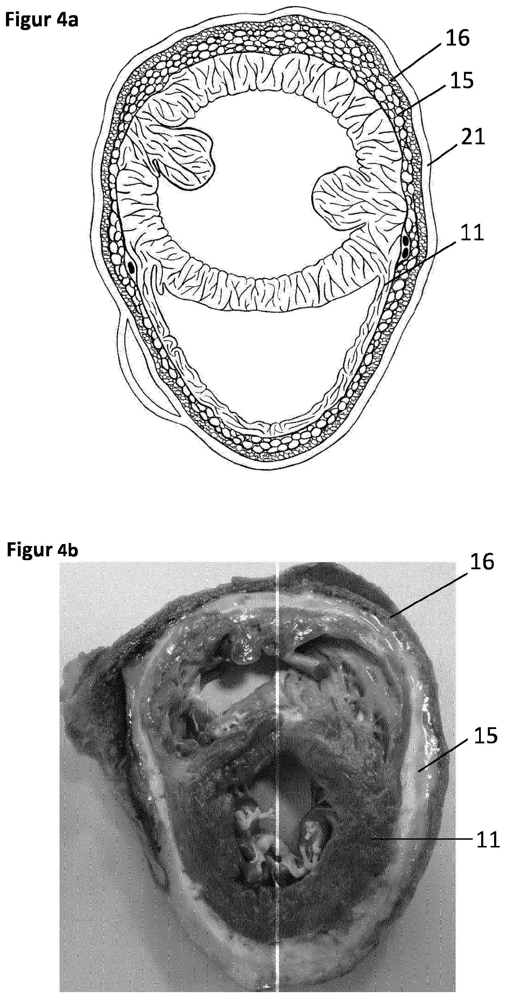

Ein Schnittbild mit Wucherungen (15, 16) um das Myokard (11) eines Herzens wie durch eine nicht biokompatible Hülle (21) ohne strukturierte Oberfläche verursacht ist schmatisch in **Figur 4a** und fotografisch in **Figur 4b** dargestellt. In den Figuren liegen herznah bereits die älteren Schichten eines abgeschlossenen Entzündungsprozesses vor (15), wohingegen in den in den äußeren, nah an der Implantatsoberfläche befindlichen Schichten die Auflagerungen (16), z.B. fibrinöse Auflagerungen (16), der chronischen und akuten Entzündungsprozesse sichtbar sind. Die sich außen anschließende Hülle (21) ist in Figur b nicht dargestellt. Vergleichbare Wucherungen (15, 16) können auch am Perikard auftreten (in den Figuren nicht dargestellt), welche durch eine nicht biokompatible Oberfläche auf der herzabgewandten, äußeren Oberfläche des Implantats verursacht werden kann.

Wucherungen (15, 16) können zu einem Verlust der Passform oder Verschiebung des Implantats führen und die natürliche Pumpfunktion des Herzens behindern. Ebenso kann dieser Prozess zu einer Abstoßung des Implantats führen oder zu einem hohen Energieverbrauch bis hin zum Funktionsverlust des Herzunterstützungssystems.

Eine poröse oder raue Strukturierung der Oberfläche der Hülle (21) führt dazu, dass die große flächenhafte Oberfläche des Implantats von den Makrophagen oder Riesenzellen nicht mehr als ununterbrochene kontinuierliche Oberfläche erkannt wird. Festkörperstrukturen zwischen den Hohlräumen einer porösen Strukturierung oder Berg-und-Tal einer rauen Strukturierung können von den Makrophagen und Riesenzellen als ausreichend kleiner Fremdkörper wahrgenommen werden, so dass eine Einkapselung und das Einwachsen von Gewebe, insbesondere von Bindegewebe, direkt an der Oberfläche des Implantats erfolgreich abgeschlossen werden kann. Die Oberfläche der Hülle (21) kann daher eine poröse oder raue Oberfläche aufweisen, bei der sich die Porengröße oder die Rauigkeit im Bereich zwischen 1 µm und 1000 µm, 10 µm bis 500 µm insbesondere zwischen 40 µm und 300 µm befindet.

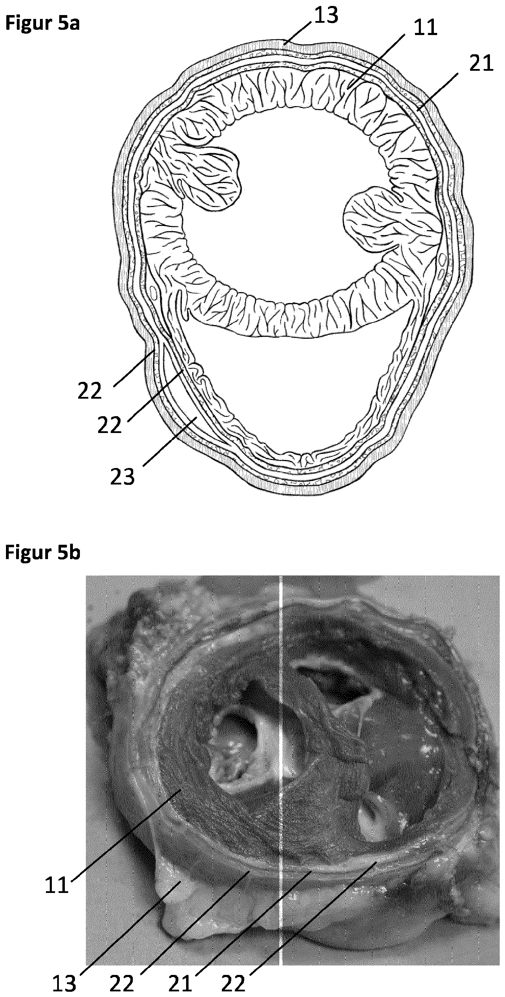

Die Oberfläche der Hülle (21) kann eine poröse oder raue Oberfläche (22) aufweisen, welche insbesondere das Einwachsen von Bindegewebe bevorzugt oder fördert. Die Rauigkeit oder Porosität sollte sich hierfür im Bereich von etwa > 40 µm bewegen. Bei Rauigkeiten oder Porosität von etwa < 40 µm wird die Zellansiedelung gegenüber einem Einwachsen mit Bindegewebe bevorzugt. Ist die gesamte Oberfläche mit Bindegewebe eingekapselt, schließen sich keine weiteren Entzündungsphasen mehr an. Der Einkapselungsprozess ist abgeschlossen. Eine mit Bindegewebe verwachsene Oberfläche der Hülle (21) ist schematisch in **Figur 5a** und fotografisch in **Figur 5b** dargestellt. Es bilden sich zwischen Epikard/Myokard (11) und Implantat und/oder zwischen Implantat und Perikard (13) keine Entzündungsschichten oder Wucherungen. Die Hülle (21) bleibt dadurch flexibel und bildet gleichzeitig ein Widerlager für das Herz und/oder expandierbare Einheiten (23).

Erfindungsgemäß kann die Hülle (21) des Implantats eine poröse Oberfläche (22) aufweisen, die sich durch Hohlräume im Kontinuum der Hülle (21) auszeichnet. Bei ausreichend hoher Porosität kann ein poröses Material von Gasen, Flüssigkeiten oder Festkörpern durchströmt werden. Porosität kann durch ein Verhältnis von Hohlräumen zu Kontinuum angegeben werden. Porosität führt in der Regel immer zu einer Abnahme der Dichte eines Werkstücks. Die Hohlräume in einem Kontinuum können geometrisch unbestimmt, unregelmäßig und von komplexer Form sein, beispielsweise bei Erzeugung durch Beflockung, Sintern oder Leaching. Bei einer Schäumung können sich regelmäßigere Geometrien ergeben, die beispielweise Kugelformen oder Ellipsoiden ähneln.

Unabhängig von der Art der Erzeugung kann Porosität über die Porengröße beschrieben werden. Die Porengröße kann angegeben werden über den größtmöglichen Durchmesser einer Kugel, die genau innerhalb eines Hohlraums einbeschrieben werden kann. Ein poröses Material kann sehr viele Hohlräume unterschiedlichster Porengröße beinhalten. Im Folgenden wird die Begrifflichkeit der "Porengröße" daher für die Größe der überwiegend vorliegenden Poren und/oder für die beabsichtigte funktionelle Porengröße verwendet. Neben dieser Porengröße können herstellbedingt noch kleinere und/oder größere Poren vorliegen, ohne dass dies im Weiteren immer gesondert angegeben wird.

Porosität kann auch mit Hilfe der Porendichte quantifiziert werden. Die Porendichte kann dabei als Anzahl von (angeschnittenen) Poren entlang einer geraden Strecke durch das Material, als Anzahl Poren in einer gedachten oder realen Schnittfläche im Material oder als Anzahl von Poren in einem definierten Volumen des Materials angegeben werden. Porosität kann auch über das Verhältnis von Hohlraumvolumen zu Gesamtvolumen eines Körpers angegeben werden.

Porengröße, Porendichte und das Hohlraum-zu-Gesamtvolumen-Verhältnis sind dabei keine unabhängigen Parameter. Sie können durch Berechnungen ineinander überführt werden. Im Folgenden wird daher vor allem die Angabe der Porengröße verwendet, womit dementsprechende Angaben zu Porendichte und Hohlraum-zu-Gesamtvolumen-Verhältnis ebenfalls offenbart sind.

Poröse Materialien können eine offene oder eine geschlossene Porosität aufweisen. Bei der offenen Porosität sind die einzelnen Poren an Kontaktstellen ohne Zwischenwand offen miteinander verbunden. Bei geschlossener Porosität sind benachbarte Poren nicht miteinander verbunden, sondern weisen zumindest eine gemeinsame Wand zwischen den Hohlräumen auf.

Bei der erfindungsgemäßen Vorrichtung kann die Hülle (21) des Implantats auch eine raue Oberfläche (22) aufweisen. Die Rauigkeit beschreibt dabei eine Oberflächenunebenheit. Eine raue Oberfläche (22) kann sich mikroskopisch als Oberfläche darstellen, bei welcher in Teilbereichen, zumeist im Bereich von kleiner 1 mm, die Oberflächenhöhe unterscheidet. Dabei kann die Rauigkeit beispielsweise in der Rautiefe, in der maximalen Rautiefe, in der gemittelten Rautiefe, im arithmetischen Mittenrauwert, in der Glättungstiefe und/oder im Profiltraganteil angegeben werden. Rauigkeiten können auch bezogen auf eine Fläche angegeben werden. Zumeist bestehen Zusammenhänge zwischen den Rauigkeitsangaben und viele Angaben können ineinander überführt werden. Im Folgenden wird daher die Rauigkeit als arithmetischer Mittenrauwert angegeben, wobei entsprechend überführte Werte für die anderen Rauigkeitsangaben gelten ohne dass diese gesondert angegeben werden.

Bei der erfindungsgemäßen Vorrichtung kann die Hülle (21) des Implantats mit oder ohne der zumindest einen expandierbaren Einheit (23) eine raue Oberfläche (22) umfassen. Bei der erfindungsgemäßen Vorrichtung kann die Hülle (21) des Implantats mit oder ohne der zumindest einen expandierbaren Einheit (23) eine kombinierte poröse und raue Oberfläche umfassen (22).

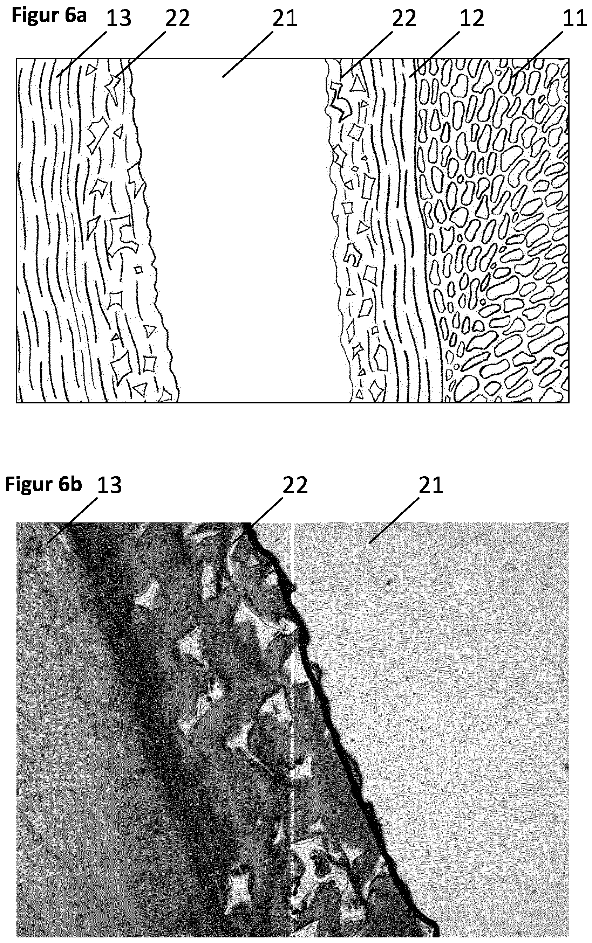

Die erfindungsgemäße poröse oder raue Oberfläche (22) fördert das Einwachsen von Gewebe, insbesondere Bindegewebe, und verhindert somit die Wucherung. **Figuren 6a und 6b** zeigen jeweils einen Schnitt durch eine erfolgreich eingekapselte Hülle (21), die auf der herzzugewandten und der herzabgewandten Seite eine poröse oder raue Oberfläche (22) aufweist. Deutlich zu erkennen ist, dass am Myokard (11) und Perikard (13) keine Wucherungen oder Entzündungen vorliegen. In Figur 6a ist schematisch dargestellt, wie unmittelbar anschließend an das Perikard (13) bzw. das Epikard (12) Gewebe in die strukturierte Oberfläche (22) eingewachsen ist. Figur 6b zeigt die Aufnahme eines histologischen Schnittes mit Perikard (13) und einer Hülle (21) in deren strukturierte Oberfläche Gewebe eingewachsen ist.

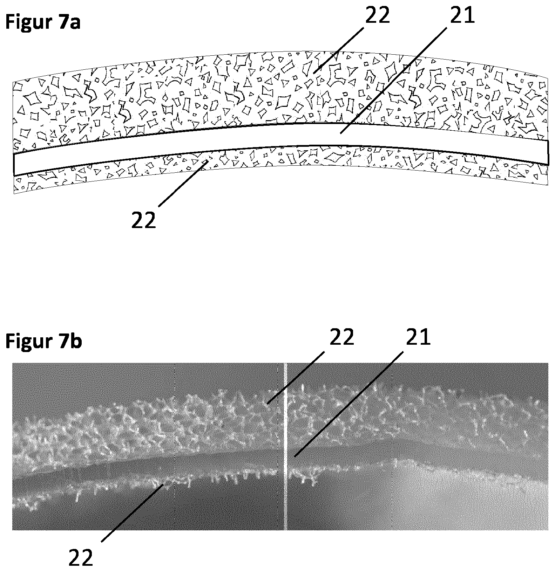

**Figur 7a** **und b** zeigen einen Schnitt durch eine erfindungsgemäße Hülle (21). Deutlich zu sehen ist, dass die obere Oberfläche der Hülle (21) eine poröse Strukturierung (22) und die untere Seite der Hülle eine raue Oberfläche (22) aufweist. Beide Oberflächenstrukturierungen (22) fördern das Einwachsen von Gewebe, insbesondere von Bindegewebe. Je nach Ausprägung der porösen bzw. rauen Oberfläche (22) kann ein zielgerichtetes Einwachsen von Bindegewebe gefördert werden. Je dicker die Strukturierungen (22) sind, desto mehr Bindegewebe wächst ein und desto länger dauert es bis das Einwachsen von Bindegewebe abgeschlossen ist. Nach dem Einwachsen ist die Hülle (21) auch an das Perikard fixiert und bildet damit ein optimales Widerlager für expandierbare Einheiten und/oder das Herz. Eine wie in Figur 1 dargestellte Schale aus einem Geflecht oder mit Streben kann damit entfallen. Es können auch nur bestimmte Bereiche einer Hülle (21) eine strukturierte Oberfläche (22) aufweisen und andere Bereiche nicht. Unterschiedliche Bereiche können unterschiedlich hoch strukturierte Oberflächen (22) aufweisen. Beispielsweise können Bereiche hinter einer expandierbaren Einheit eine höhere Strukturierung (22) aufweisen, beispielsweise um ein besseres mechanisches Widerlager darzustellen. Weiter können Bereiche hinter oder vor expandierbaren Einheiten oder Elektroden eine höhere Strukturierung (22) aufweisen, um mehr Gewebe in die strukturierte Oberfläche (22) einwachsen zu lassen, womit ein besserer Schutz vor Verletzungen des Epi-/Peri- und/oder Myokards sowie umgebender Strukturen oder Organe durch die Elektroden oder Elemente einer expandierbaren Einheit erreicht wird. Weiter können Bereiche hinter oder vor expandierbaren Einheiten oder Elektroden eine weniger hohe Strukturierung aufweisen, um der Dicke dieser Elemente Rechnung zu tragen und die von diesen verursachten Unebenheiten auf der Implantatsoberfläche auszugleichen.

Es kann so durch die unterschiedlich hohe Strukturierung (22) eine ebene Oberfläche des Implantats erreicht werden.

Eine Hülle (21) mit poröser oder rauer Oberfläche (22) kann aus den verschiedensten Materialien hergestellt werden. Die poröse oder raue Oberfläche (22) kann durch Aufbringen einer Beschichtung erzielt werden, wobei die aufgebrachte Beschichtung aus dem gleichen oder einem anderen Material wie die Hülle (21) besteht. Die poröse oder raue Oberfläche (22) kann auch durch Umwandeln des Materials der Hülle (21) erzielt werden und besteht dann aus dem gleichen bzw. modifizierten Material. Als Material für die Hülle (21) und/oder die aufgebrachte Beschichtung kommen unter anderem PLA, PGA, PCL, PLLA, PLGA, PE, PTFE, ePTFE, Polyester (z.B. PET), Silikon, Polyurethan und zahlreiche andere Kunststoffe oder Spider Silk in Frage.

Im Folgenden werden verschiedene Verfahren zur Herstellung eines Implantats umfassend eine Hülle mit einer porösen oder rauen Oberfläche beschrieben. Analog gelten die nachfolgenden Beschreibungen zur Herstellung einer Hülle mit einer strukturierten Oberfläche auch für die Herstellung einer expandierbaren Einheit mit strukturierter Oberfläche. Eine Hülle und/oder eine expandierbare Einheit mit poröser oder rauer Oberfläche kann urformend, durch Aufbringen einer porösen oder rauen Beschichtung auf ein Kernmaterial einer Hülle und/oder einer expandierbaren Einheit oder durch Oberflächenumwandlung einer nicht strukturierten Oberfläche erfolgen.

Die poröse oder raue Oberfläche kann bereits im Rohmaterial für die Hülle vorhanden sein oder kann während der Fertigung erstellt werden oder kann im Anschluss an die Formgebung der Hülle hinzugefügt werden, beispielsweise in Form einer Beschichtung.

Materialien die von sich aus eine poröse oder raue Oberfläche haben umfassen unter anderem geschäumte Materialen. Diese können je nach Herstellungsverfahren eine unterschiedliche Porosität oder Rauigkeit aufweisen. Das geschäumte Material kann bei der Herstellung mit einem gasbildenden Reagenz versetzt werden. Beispielsweise kann Wasser, das während des Prozesses zugegeben wird, mit funktionellen Gruppen eines Kunststoffes zu einem Gas (z.B. CO₂) reagieren, dessen Austritt zu Schaumbildung und somit zu einer Oberflächenstrukturierung führt. Eine Alternative zur Herstellung eines schaumartigen Materials umfasst den 3D-Druck des Schaumes. Eine weitere Alternative zur Herstellung eines schaumartigen Materials umfasst das "Leaching"-Verfahren. Bei diesen Verfahren werden Stoffe zugegeben, die nach Polymerisation des Kunststoffes wieder herausgelöst werden, beispielsweise durch Kristallisation, durch Auflösen mit Hilfe eines geeigneten Lösemittels oder durch thermisches Ausschmelzen.

Ein weiteres Material, das von sich aus eine poröse oder raue Oberfläche umfasst, sind Faserwerkstoffe. Faserwerkstoffe können beispielsweise durch Weben, Filzen, Flechten oder andere Verfahren hergestellt werden. Damit weisen auch Hüllen aus diesen Materialien eine Oberfläche mit einer Porosität oder Rauigkeit auf. Eine erfindungsgemäße Hülle kann aus einem Faserwerkstoff durch umformen (z.B. Formen unter Temperatureinwirkung) oder durch Nähen hergestellt werden. Die erfindungsgemäße Hülle kann auch dadurch hergestellt werden, dass solche Faserwerkstoffeals strukturierte Oberfläche auf ein Kernmaterial einer Hülle beschichtet werden.

Weitere Materialien, die von sich aus poröse oder raue Oberfläche umfassen, können auch aus kleinen Festkörpern gepackte Schichten (vgl. "Sintern" oder Beflocken) oder eine Wabenstruktur sein.

Materialien, die nicht per se eine poröse oder raue Oberfläche haben, können durch Oberflächenmodifikation, beispielsweise durch Entfernen von Material aus der Hülle eine poröse oder raue Oberfläche bekommen. Geeignete Verfahren zum Herauslösen von Material umfassen chemische Verfahren (z.B. "Leaching", Ätzverfahren, Schäumverfahren etc.) oder physikalische Verfahren (z.B. Bohren, Laserbohren, Bestrahlen, Erodieren, Sandstrahlen, Einprägen, Einwalzen, etc.).

Es ist auch möglich, im Zuge der Formgebung der Hülle eine raue oder poröse Oberfläche zu schaffen. Die Hülle kann beispielsweise durch Tauchen oder Gießen geformt werden. Hierfür kann eine Positivform eines Herzens zumindest einmal in eine Lösung aus flüssigem Kunststoff (z.B. Silikon, Polyurethan, Polyester) getaucht oder mit einer Lösung aus flüssigem Kunststoff übergossen werden. Mit der Anzahl der Tauch- oder Gießvorgänge kann die Schichtdicke des Hüllenmaterials eingestellt werden. Weniger Tauch- oder Gießvorgänge führen zu dünneren Hüllen, mehrere Tauch- und Gießvorgänge führen zu dickeren Hüllen.

Zwischen den Tauch- oder Gießvorgängen können Pausenzeiten zur Trocknung und Anbindung des neu aufgebrachten Kunststoffs (Polymerisation) erforderlich sein. Eine Strukturierung der Oberfläche kann beispielsweise dadurch erreicht werden, dass strukturierende Elemente (z.B. Flocken, Kugeln, Kristalle, geometrisch definierte oder undefinierte Körper, Schäume und/oder Kugelsegmente) auf eine getauchte oder gegossene Hülle aufgebracht werden, dessen äußere Kunststoffschichten noch nicht vollständig polymerisiert sind.

Eine Hülle mit strukturierter Oberfläche, welche ein Einwachsen von Gewebe fördert, kann beispielsweise urformend hergestellt werden. Bei der gusstechnischen Herstellung einer Hülle (inklusive Spritzguss, Tauchverfahren, Vulkanisieren, etc.).

Eine raue oder poröse Oberfläche kann beispielsweise auch dadurch erzielt werden, dass die Oberfläche der verwendeten Gussform selbst Unebenheiten aufweist. Beispielsweise können Oberflächenunebenheiten und/oder Strukturierungen durch Ätzen, Bohren, Laserbohren, Bestrahlen, Erodieren, Sandstrahlen, Einprägen, Einwalzen, Aufrauen, Schleifen oder durch Fräsen auf der Oberfläche der Gussform erzeugt werden. Wird die Hülle nach dem Gießen von der Form befreit, weist die Hülle das entsprechende Negativ der Oberflächenunebenheit/- strukturierung der Gussform als strukturierte Oberfläche der Hülle auf.

Alternativ oder zusätzlich kann ein strukturierendes Element mit rauer und/oder poröser Oberfläche auf der Gussform angebracht werden, beispielsweise Strukturpapier, Mattierungsfolie oder strukturierte Folie. Nach dem Gießen der Hülle kann das strukturierende Element von der Hülle abgezogen werden, wobei dieses eine entsprechende strukturierte Oberfläche auf der Hülle hinterlässt.

Alternativ oder zusätzlich kann eine strukturierte Oberfläche einer Hülle, welche das Einwachsen von Gewebe fördert, durch eine raue oder poröse Beschichtung, welche auf die Hülle aufgebracht wird, realisiert werden. Eine auf eine Hülle aufgebrachte raue oder poröse Beschichtung kann vor dem Anbringen in einer ebenen Form, beispielweise als Tuch, Folie oder in Plattenform, jeweils beispielsweise in einer Dicke von 0.1 mm bis 10 mm, 0.5 mm bis 5 mm, insbesondere zwischen 1.5 mm und 4 mm vorliegen, elastisch/verformbar sein und über die Hülle gezogen/gespannt werden.

Eine auf eine Hülle aufgebrachte raue oder poröse Beschichtung kann vor dem Anbringen auch in einer runden, zylindrischen, beispielweise als Schlauch oder Rohr, jeweils beispielsweise in einer Wandstärke von 0.1 mm bis 10 mm, 0.5 mm bis 5 mm, insbesondere zwischen 1.5 mm und 4 mm vorliegen, elastisch/verformbar sein und über die Hülle gezogen/gespannt werden. Die Beschichtung kann beispielsweise auf eine getauchte oder gegossene Hülle aufgebracht werden, dessen äußere Kunststoffschichten noch nicht vollständig polymerisiert sind. Die Beschichtung wird dann bis zur vollständigen Polymerisation der Hülle auf diese gespannt/gepresst und somit mit dieser verbunden werden. Mit diesem Verfahren erhält man eine stabile Verbindung zwischen der aufgebrachten Beschichtung und der Hülle, die sich auch unter Belastung nicht löst. Die Verbindung zwischen der Hülle und der Beschichtung kann stoffschlüssig und/oder formschlüssig erfolgen.

Bei diesem Prozess kann die Dicke der ebenen Beschichtung durch das Ziehen über die Hülle und durch das Eindringen in die noch nicht vollständig polymerisierte Kunststoffmasse effektiv reduziert werden, so dass etwas geringere Dicken als beim Ausgangsmaterial erreicht werden. Weiter können bei dieser Methode die Geometrien der Hohlräume etwas entarten. Beispielsweise können runde Hohlräume durch das Ziehen über die Hülle/die Form gestreckt werden, so dass am Ende gestreckte Hohlräume vorliegen. Im Falle kugelförmiger Hohlräume würden dann ellipsoide Hohlräume durch die Fertigung entstehen. Weiter können durch diese Fertigungsmethode durch die Verformung der Beschichtung bei der Verbindung Spannungen in der Hülle und in der Beschichtung entstehen, die im gefertigten Zustand Verformungen der Hülle und der Beschichtung verursachen, so dass die Vorrichtung entartet und nicht mehr der ursprünglichen Herzform entspricht. In diesem Falle kann es beispielsweise erforderlich sein, dass eine Wärmebehandlung der Hülle mit der Beschichtung auf der Positivform erforderlich ist, so dass eventuelle Spannungen reduziert oder abgebaut werden ("annealing", "Tempern", "Normalisieren", "Spannungsarmglühen"). Die Porengröße/Rauheit des Ausgangsmaterials der Beschichtung kann so gewählt werden, dass die Porengröße nach der Fertigung der angestrebten Porengröße entspricht.

Weiter kann beispielsweise ein Thermoform- oder Tiefziehprozess an der aufzubringenden Beschichtung erfolgen, bevor diese mit der Hülle verbunden wird. Eine vorgeformte Beschichtung kann so spannungsfrei mit der Hülle verbunden werden, ohne dass Spannungen entstehen, welche den Verbund aus Hülle und Beschichtung entarten lassen.

Ist die Hülle vor dem Anbringen der Beschichtung bereits vollständig polymerisiert, kann auch ein Kleber auf die Hülle oder eine vorgeformte oder nicht vorgeformte Beschichtung aufgebracht werden. Die Hülle und die Beschichtung können somit über einen Klebeprozess miteinander verbunden werden. Der Kleber kann auch ein Schmelzkleber sein, so dass Hülle und Beschichtung zunächst einfach nur in Kontakt zueinander gebracht werden und die Aktivierung der Verklebung durch Erwärmung der in Kontakt stehenden Elemente erfolgt.

Die Hülle kann auch mittels Thermoformen hergestellt werden. Beim Thermoformen kann ein thermoplastischer Kunststoff unter Wärmeeinwirkung mit Hilfe einer Positiv- oder NegativForm eines Herzabbildes in die gewünschte Zielform gebracht werden. Das Rohmaterial kann in flacher oder zylindrischer Form vorliegen, beispielsweise als Folie, Tuch, Platte, oder als schlauch- bzw. rohrartiges Halbzeug vorliegen. Bei Verwendung einer Negativform des Herzabbildes kann erwärmtes Material mittels Unterdruck oder Vakuum in die Form gezogen werden. Die Negativform bildet so die Außenkontur/die herzabgewandte Seite der Hülle ab. Bei Verwendung einer Positivform des Herzabbildes kann erwärmtes Material mit oder ohne Unterdruck/Vakuum über die Form gezogen werden. Die Positivform bildet so die Innenkontur/die herzzugewandte Seite der Hülle ab. Sowohl bei der Verwendung einer Positivform wie auch bei einer Negativform kann die Oberfläche der Form derart sein, dass während des Thermoformens eine Strukturierung im Kunststoff der Hülle erzeugt wird. Beispielsweise kann die Form eine aufgeraute Oberfläche besitzen, welche eine Rauheit oder Porosität von ungefähr > 40 µm aufweist. Die Oberfläche kann sich in entsprechender Strukturierung auf der gefertigten Hülle abbilden.

Die poröse oder raue Beschichtung kann auch durch thermische Fügeverfahren an die Hülle verbunden werden. Beispielsweise kann eine noch nicht an das Implantat angebundene Beschichtung vor dem Thermoformen der Hülle in oder über die Negativ-/Positivform angebracht werden. Wird dann das erhitzte Material der Hülle im Thermoformprozess in oder über die Form gezogen, so kann sich die Beschichtung mit dem angeschmolzenen Material der Hülle verbinden.

Eine bereits geformte Hülle kann auch nochmal an seiner Oberfläche erwärmt und/oder angeschmolzen werden und eine raue oder poröse Beschichtung kann dann, beispielsweise durch unterstützendes Andrücken, thermisch mit dem Kernmaterial der Hülle verbunden werden. Die Oberfläche einer Hülle oder einer Beschichtung kann zu diesem Zweck auch mit einem Schmelzkleber versehen sein oder mit einem Kunststoff, der einen niedrigeren Schmelzpunkt aufweist als das Kernmaterial der Hülle oder das Material der Beschichtung

Eine Hülle mit strukturierter Oberfläche, welche ein Einwachsen von Gewebe fördert, kann alternativ oder zusätzlich durch Oberflächenumwandlung hergestellt werden.

Wird eine Hülle beispielsweise gusstechnisch hergestellt, kann ein Opfermaterial auf die Oberfläche der Gussform aufgebracht werden, welches nach dem Gießen der Hülle wieder entfernt wird. Ein einfaches Beispiel für ein Opfermaterial sind Salzkristalle. Werden diese auf die Gussform vor dem Gießen aufgebracht und nach dem Gießen der Hülle beispielsweise mit Wasser ausgewaschen, so entsteht eine poröse oder raue Oberfläche. Durch Auswahl einer geeigneten Körnung der Salzkristalle kann eine geeignete Porosität oder Rauigkeit auf der Hülle erzeugt werden.

Zusätzlich oder alternativ können Strukturierungsadditive, z.B. Additive zur Schäumung oder Mattierungsadditive, einem Rohmaterial und/oder einem Halbzeug zugegeben werden. Das Rohmaterial und/oder Halbzeug kann dann der Formgebung (urformend oder umformend) unterzogen werden. Nach der Formgebung können dann die Additive über chemische oder physikalische Mechanismen (z.B. Leaching, Auflösen, Quellen, Schmelzen, ...) aktiviert werden, wodurch durch chemische Reaktion oder über mechanische Mechanismen (z.B. Aufreißen der Oberfläche durch ausdringende Reaktionsprodukte von Additiven) eine strukturierte Oberfläche erzeugt wird.

Eine gefertigte Hülle kann durch Perforieren oder Bohren, beispielsweise Laserbohren, an der Oberfläche strukturiert werden um im Anschluss an einen Guss-, Tauch- oder Thermoformprozess eine Rauigkeit oder Porosität von beispielsweise ungefähr > 40 µm zu erzeugen.

Das Material einer Hülle kann auch nur teilweise strukturiert werden. Beispielsweise können Bereiche, unter welcher sich eine expandierbare Einheit befindet nicht gebohrt oder perforiert werden, um damit Undichtigkeiten zu vermeiden, die einen Austausch von Fluiden über die Implantatsgrenzen mit dem Körper oder Körperflüssigkeiten erzeugen könnten. Weiter kann sich die Strukturierung in unterschiedlicher Tiefe darstellen. Perforationen oder Bohrungen, bzw. Strukturierungen im Allgemeinen, können durch das gesamte Material der Hülle gehen oder können nur teilweise in das Material gehen. Beispielsweise bietet es sich an, dass Strukturierungen der zumindest einen expandierbaren Einheit oder in Bereichen der Hülle unter welchen sich ein expandierbare Einheit befindet nur teilweise in das Kernmaterial der Hülle übergehen. Beispielsweise können Strukturierungen/Bohrungen/Perforationen nur bis zur Hälfte der Tiefe des Kernmaterials gehen oder nur weniger als 200 µm, weniger als 100 µm oder weniger als 50 µm tief sein. So können Undichtigkeiten vermieden werden oder auch die Lebensdauer des Implantats / der Hülle / der zumindest einen expandierbaren Einheit hochgehalten werden.

Verschiedene Bereiche einer Hülle können mit gleichen oder unterschiedlichen Verfahren strukturiert werden. Beispielsweise können die herzzugewandte Seite und die herzabgewandte Seite einer Hülle mit Hilfe gleicher oder unterschiedlicher Verfahren mit einer porösen oder rauen Strukturierung versehen werden. Beispielsweise kann eine Strukturierung auf einer Seite urformend hergestellt werden und auf der anderen Seite durch Hinzufügen einer Beschichtung. Beispielsweise kann eine Schicht auf einer Seite durch Beschichtung und auf der anderen Seite durch Oberflächenumwandlung erzeugt werden. Beispielsweise kann eine Beschichtung auf einer Seite thermisch gefügt werden und auf der anderen Seite angeklebt werden.

Als Material für die Hülle (21) und/oder die aufgebrachte Beschichtung kommen unter anderem PLA, PGA, PCL, PLLA, PLGA, PE, PTFE, ePTFE, Polyester (z.B. PET), Silikon, Polyurethan und zahlreiche andere Kunststoffe oder Spider Silk in Frage.

Bei den Polyurethanen bieten sich Polyesterurethane, Polyetherurethane und insbesondere Polycarbonaturethane an.

Polyesterurethane sind stabil gegen oxidative Degradation und haben gute mechanische Eigenschaften (hohe Festigkeit, abriebsfest), sind aber anfällig gegen hydrolytische Spaltung, was v.a. für Implantationen in feuchter Umgebung, beispielsweise in den menschlichen Körper nachteilig ist.

Polyetherurethane sind stabiler gegen hydrolytische Spaltung, wofür sie für den Einsatz in der feuchten Körperumgebung besser geeignet sind als Polyesterurethane, sind aber anfällig gegen oxidative Degradation.

Polycarbonaturethane sind stabiler gegen hydrolytische Spaltung als Polyetherurethane und stabiler gegen oxidative Degradation als Polyesterurethane. Polycarbonaturethane können daher für eine dauerhafte Implantation in feuchter Umgebung besser geeignet sein als Polyesterurethane oder Polyetherurethane und bei hydrolytischer, oxidative, enzymatischer und mechanischer Belastung größere Lebensdauern erreichen. Beispiele für Polycarbonaturethane sind DSM Bionate^{®} PCU, DSM Bionate^{®} II PCU, Lubrizol Carbothane^{™} TPU (aliphatisch, aromatisch).

## Patentansprüche

1. Herzunterstützungsvorrichtung umfassend ein Implantat mit einer Hülle (21), wobei die Hülle (21) eine innere Oberfläche (22) und eine äußere Oberfläche (22) hat,
die innere Oberfläche (22) herzzugewandt ist und ausgelegt ist, im implantierten Zustand das Epikard (12) zu berühren,
die äußere Oberfläche (22) herzabgewandt ist und ausgelegt ist, im implantierten Zustand das Perikard (13) zu berühren,
**dadurch gekennzeichnet, dass** die innere und die äußere Oberfläche (22) der Hülle (21) eine strukturierte Oberfläche (22) hat, die das Einwachsen von Bindegewebe fördert.

2. Herzunterstützungsvorrichtung nach Anspruch 1, wobei die Oberfläche (22) der Hülle (21) eine poröse Oberfläche (22) umfasst.

3. Herzunterstützungsvorrichtung nach Anspruch 1 oder 2, wobei die Oberfläche (22) der Hülle (21) Poren umfasst, wobei die Poren eine Größe von 1 µm bis 1000 µm, insbesondere von 10 µm bis 500 µm umfassen.

4. Herzunterstützungsvorrichtung nach Anspruch 3, wobei die Poren eine Größe von 40 µm bis 300 µm haben.

5. Herzunterstützungsvorrichtung nach Anspruch 1, wobei die Oberfläche (22) der Hülle (21) eine raue Oberfläche (22) umfasst.

6. Herzunterstützungsvorrichtung nach einem der vorhergehenden Ansprüche, wobei die Oberfläche (22) der Hülle (21) eine Oberflächenbeschichtung umfasst.

7. Herzunterstützungsvorrichtung nach Anspruch 6, wobei die Oberflächenbeschichtung der Hülle (21) eine poröses Material umfasst.

8. Herzunterstützungsvorrichtung nach Anspruch 7, wobei das poröse Material eine geschäumte Schicht umfasst.

9. Herzunterstützungsvorrichtung nach Anspruch 7, wobei das poröse Material Fasern umfasst.

10. Herzunterstützungsvorrichtung nach einem der Ansprüche 1 bis 5, wobei die strukturierte Oberfläche (22) der Hülle (21) durch eine Oberflächenumwandlung erzielt wird.

11. Herzunterstützungsvorrichtung nach Anspruch 10, wobei die Oberflächenumwandlung der Hülle (21) eine poröse Oberfläche (22) erzeugt.

12. Herzunterstützungsvorrichtung nach einem der vorhergehenden Ansprüche, wobei die Herzunterstützungsvorrichtung eine aktive Herzunterstützungsvorrichtung ist.

13. Herzunterstützungsvorrichtung nach Anspruch 12, wobei die aktive Herzunterstützungsvorrichtung zumindest eine expandierbare Einheit (23) umfasst.

14. Herzunterstützungsvorrichtung nach einem der Ansprüche 1 bis 11, wobei die Herzunterstützungsvorrichtung eine passive Herzunterstützungsvorrichtung ist.

15. Verfahren zum Herstellen einer Herzunterstützungsvorrichtung umfassend:
Bereitstellen eines Implantats mit einer Hülle (21), wobei die Hülle (21) eine innere und eine äußere Oberfläche (22) hat, wobei die innere Oberfläche (22) herzzugewandt ist und ausgelegt ist, im implantierten Zustand das Epikard (12) zu berühren, wobei die äußere Oberfläche (22) herzabgewandt ist und ausgelegt ist, im implantierten Zustand das Perikard (13) zu berühren, **gekennzeichnet durch**
Anbringen einer porösen oder rauen Beschichtung auf die innere und die äußere Oberfläche (22) der Hülle (21), wobei die Beschichtung das Einwachsen von Bindegewebe fördert; oder
Umwandeln der inneren und der äußeren Oberfläche (22) der Hülle (21), sodass eine poröse oder raue Oberfläche (22) entsteht, die das Einwachsen von Bindegewebe fördert.

## Claims

1. Heart assist device comprising an implant having a sheath (21), wherein the sheath (21) has an inner surface (22) and an outer surface (22),
the inner surface (22) faces towards the heart and is configured to touch the epicardium (12) when implanted,
the outer surface (22) faces away from the heart and is configured to touch the pericardium (13) when implanted,
**characterized in that** the inner and the outer surface (22) of the sheath (21) has a structured surface (22) which promotes the ingrowth of connective tissue.

2. Heart assist device according to Claim 1, wherein the surface (22) of the sheath (21) comprises a porous surface (22).

3. Heart assist device according to Claim 1 or 2, wherein the surface (22) of the sheath (21) comprises pores, wherein the pores have a size of 1 µm to 1000 µm, in particular from 10 µm to 500 µm.

4. Heart assist device according to Claim 3, wherein the pores have a size of 40 µm to 300 µm.

5. Heart assist device according to Claim 1, wherein the surface (22) of the sheath (21) comprises a rough surface (22).

6. Heart assist device according to any of the preceding claims, wherein the surface (22) of the sheath (21) comprises a surface coating.

7. Heart assist device according to Claim 6, wherein the surface coating of the sheath (21) comprises a porous material.

8. Heart assist device according to Claim 7, wherein the porous material comprises a foamed layer.

9. Heart assist device according to Claim 7, wherein the porous material comprises fibres.

10. Heart assist device according to any of Claims 1 to 5, wherein the structured surface (22) of the sheath (21) is obtained by a surface conversion.

11. Heart assist device according to Claim 10, wherein the surface conversion of the sheath (21) generates a porous surface (22).

12. Heart assist device according to any of the preceding claims, wherein the heart assist device is an active heart assist device.

13. Heart assist device according to Claim 12, wherein the active heart assist device comprises at least one expandable unit (23).

14. Heart assist device according to any of Claims 1 to 11, wherein the heart assist device is a passive heart assist device.

15. Method for producing a heart assist device comprising:
providing an implant having a sheath (21), wherein the sheath (21) has an inner and an outer surface (22), wherein the inner surface (22) faces towards the heart and is configured to touch the epicardium (12) when implanted, wherein the outer surface (22) faces away from the heart and is configured to touch the pericardium (13) when implanted, **characterized by**
application of a porous or rough coating to the inner and the outer surface (22) of the sheath (21), wherein the coating promotes the ingrowth of connective tissue; or
conversion of the inner and the outer surface (22) of the sheath (21), such that a porous or rough surface (22) which promotes the ingrowth of connective tissue is formed.

## Revendications

1. Dispositif d'assistance cardiaque comprenant un implant avec une enveloppe (21), l'enveloppe (21) présentant une surface intérieure (22) et une surface extérieure (22),
la surface intérieure (22) étant tournée vers le cœur et étant conçue pour entrer en contact avec l'épicarde (12) à l'état implanté,
la surface extérieure (22) étant détournée du cœur et étant conçue pour entrer en contact avec le péricarde (13) à l'état implanté,
**caractérisé en ce que** les surfaces intérieure et extérieure (22) de l'enveloppe (21) présentent une surface structurée (22) qui favorise la croissance de tissu conjonctif.

2. Dispositif d'assistance cardiaque selon la revendication 1, où la surface (22) de l'enveloppe (21) comprend une surface poreuse (22).

3. Dispositif d'assistance cardiaque selon la revendication 1 ou 2, où la surface (22) de l'enveloppe (21) comprend des pores, les pores ayant une taille de 1 µm à 1000 µm, notamment de 10 µm à 500 µm.

4. Dispositif d'assistance cardiaque selon la revendication 3, où les pores ont une taille de 40 µm à 300 µm.

5. Dispositif d'assistance cardiaque selon la revendication 1, où la surface (22) de l'enveloppe (21) comprend une surface rugueuse (22).

6. Dispositif d'assistance cardiaque selon l'une quelconque des revendications précédentes, où la surface (22) de l'enveloppe (21) comprend un revêtement de surface.

7. Dispositif d'assistance cardiaque selon la revendication 6, où le revêtement de surface de l'enveloppe (21) comprend un matériau poreux.

8. Dispositif d'assistance cardiaque selon la revendication 7, où le matériau poreux comprend une couche expansée.

9. Dispositif d'assistance cardiaque selon la revendication 7, où le matériau poreux comprend des fibres.

10. Dispositif d'assistance cardiaque selon l'une quelconque des revendications 1 à 5, où la surface structurée (22) de l'enveloppe (21) est obtenue par une transformation de surface.

11. Dispositif d'assistance cardiaque selon la revendication 10, où la transformation de surface de l'enveloppe (21) produit une surface poreuse (22).

12. Dispositif d'assistance cardiaque selon l'une quelconque des revendications précédentes, où le dispositif d'assistance cardiaque est un dispositif d'assistance cardiaque actif.

13. Dispositif d'assistance cardiaque selon la revendication 12, où le dispositif d'assistance cardiaque actif comprend au moins une unité expansible (23).

14. Dispositif d'assistance cardiaque selon l'une quelconque des revendications 1 à 11, où le dispositif d'assistance cardiaque est un dispositif d'assistance cardiaque passif.

15. Procédé de fabrication d'un dispositif d'assistance cardiaque comprenant :
la fourniture d'un implant avec une enveloppe (21), l'enveloppe (21) présentant une surface intérieure et une surface extérieure (22), la surface intérieure (22) étant tournée vers le cœur et conçue pour entrer en contact avec l'épicarde (12) à l'état implanté, la surface extérieure (22) étant détournée du cœur et conçue pour pour entrer en contact avec le péricarde (13) à l'état implanté, **caractérisé par**
l'application d'un revêtement poreux ou rugueux sur les surfaces intérieure et extérieure (22) de l'enveloppe (21), ledit revêtement favorisant la croissance de tissu conjonctif ; ou
la transformation des surfaces intérieure et extérieure (22) de l'enveloppe (21) afin d'obtenir une surface poreuse ou rugueuse (22) favorisant la croissance de tissu conjonctif.
